# EUROPÄISCHE PATENTANMELDUNG

(11) **EP 2 223 747 A1**
(43) Veröffentlichungstag der Anmeldung: **01.09.2010**
(21) Anmeldenummer: 09152982.6
(22) Anmeldetag: 17.02.2009
(51) Int. Cl.: B01L 3/02, A61B 5/151

(54) **Verfahren und Vorrichtung zur Bereitstellung von Blutbestandteilen**

(71) Anmelder: Roche Diagnostics GmbH, 68305 Mannheim (DE); F. Hoffmann-La Roche AG, 4070 Basel (CH)
(72) Erfinder: Die Erfindernennung liegt noch nicht vor
(74) Vertreter: Stößel, Matthias

(57) **Zusammenfassung**

Es wird ein Verfahren zur Bereitstellung mindestens eines definierten Volumens eines Zielbestandteils (164) einer Probe (134) vorgeschlagen. Das Verfahren umfasst die folgenden Schritte:
- Bereitstellen mindestens einer Dosierkapillare (110) mit mindestens zwei Öffnungen (112, 114);
- zumindest teilweises Befüllen der Dosierkapillare (110) mit der Probe (134);
- Durchführen einer Bestandteilsseparation zur zumindest teilweisen Trennung mindestens zweier Bestandteile (150, 154) der Probe (134) innerhalb der Dosierkapillare (110); und
- Aufteilung der Dosierkapillare (110) in mindestens zwei Teilstücke (160, 162), wobei mindestens eines der Teilstücke (160) das definierte Volumen des Zielbestandteils (164) enthält.

## Beschreibung

### Gebiet der Erfindung

Die Erfindung betrifft ein Verfahren zur Bereitstellung mindestens eines definierten Volumens eines Zielbestandteils einer Probe, insbesondere einer Blutprobe. Weiterhin betrifft die Erfindung eine Dosierkapillare sowie eine Vorrichtung zur Bereitstellung mindestens eines definierten Volumens eines Zielbestandteils einer Probe. Derartige Verfahren, Dosierkapillaren und Vorrichtungen können insbesondere zur Gewinnung definierter Plasmamengen aus Kapillarblut eingesetzt werden.

### Stand der Technik

Die Untersuchung von Proben von Körperflüssigkeiten, wie beispielsweise Blut, bildet oft einen wesentlichen Bestandteil der medizinischen Diagnostik. Derartige Untersuchungen können sowohl im Klinikbereich als auch im Point-of-Care-Bereich oder im Bereich des Home-Monitorings eingesetzt werden. Die nachfolgend beschriebene Erfindung wird insbesondere unter Bezugnahme auf Blutproben dargestellt, wobei jedoch sinngemäß auch andere Arten von Proben, insbesondere flüssige Proben und vorzugsweise Körperflüssigkeiten, untersucht werden können. Ohne Beschränkung weiterer möglicher Arten von Proben wird die Erfindung im Folgenden im Wesentlichen unter Bezugnahme auf Blutproben beschrieben.

Die Proben werden in der Regel mindestens einer medizinischen und/oder diagnostischen Verwendung zugeführt. Bei einer diagnostischen Verwendung können diese beispielsweise auf mindestens eine Eigenschaft untersucht werden, beispielsweise mindestens einen physikalisch und/oder chemisch bzw. biochemisch messbaren Parameter. Beispielsweise können die Proben einem qualitativen und/oder quantitativen Nachweis mindestens eines Analyten, insbesondere mindestens eines Metaboliten, unterzogen werden. Zu diesem Zweck sind aus dem Stand der Technik zahlreiche Nachweisverfahren bekannt.

Beispielsweise können Glukose, Cholesterin, Triglyceride, Hämoglobin, Harnstoff, Alanin-Aminotransferase (ALT), Aspartat-Aminotransferase (AST), Gamma-Glutamyltransferase (GGT), Creatinin (CREA) oder High-density-Lipoprotein (HDL) oder Kombinationen der genannten Analyte oder anderer Analyte nachgewiesen werden. Daneben können andere Eigenschaften des Blutes bestimmt werden, wie beispielsweise ein Anteil korpuskulärer Bestandteile (Hämatokrit-Wert).

Eine Problematik bei der Untersuchung beispielsweise von Blutproben, aber auch von anderen Arten von Proben, besteht darin, dass diese Blutproben in vielen Fällen vor der weiteren Verwendung aufbereitet werden müssen. Insbesondere ist es für zahlreiche Anwendungen erforderlich, Blutproben in ihre Bestandteile zu zerlegen und beispielsweise Blutplasma von korpuskulären Bestandteilen der Blutprobe zu trennen. Diese Trennung der Blutprobe in ihre Bestandteile muss in der Regel sehr sorgfältig durchgeführt werden, da für viele Messungen eine hohe Reinheit, d.h. ein hoher Trennungsgrad, bei gleichzeitig exakt definierten Probenmengen erforderlich ist. Diese hohen Anforderungen hinsichtlich der Präzision der Probenvorbereitung erschweren oder verhindern in vielen Fällen eine Realisierung von Analysen durch Laien, da die Probenpräparation zur Gewährleistung der genannten Anforderungen in der Regel durch geschultes Personal durchgeführt werden muss. Beispielsweise sind bislang keine oder nur wenige Vorrichtungen auf dem Markt, mittels derer der Parameter HDL-C, (High Density-Lipoprotein, Variante C) gemessen werden kann. Dies ist insbesondere dadurch begründet, dass dieser Parameter wegen der bei der Abtrennung auftretenden Gerinnung ausschließlich in mit Antikoagulanzien versetztem Plasma bei exakt definiertem Probenvolumen (beispielsweise 31 ± 1,5 µl) gemessen werden kann, also erst nach sehr sorgfältiger Probenvorbereitung.

Eine bekannte Methode zur Gewinnung von Plasma aus Blutproben, beispielsweise Kapillarplasma, ist eine Gewinnung von Kapillarblut mit anschließender Zentrifugation. So kann beispielsweise aus einem Einstich in einer Körperoberfläche mittels einer Kapillare austretendes Blut aufgenommen werden, um dieses anschließend einer Zentrifugation zu unterziehen. Mittlerweile sind kommerziell auch Kapillaren erhältlich, welche nach dem Befüllen an einer Sollbruchstelle gebrochen werden, um ein exaktes Probenvolumen zu erhalten. Derartige Kapillaren sind beispielsweise kommerziell von Dr. Müller Gerätebau GmbH in D-01705 Freital, Deutschland erhältlich oder sind beispielsweise in DE 295 20 918 U1 beschrieben. Die gebrochenen Kapillarteile mit der darin befindlichen Menge an Blut werden anschließend in ein Probengefäß eingegeben, beispielsweise ein Spitzbodengefäß (Cup). Derartige Probengefäße werden dann in entsprechenden Zentrifugen zentrifugiert. Alternativ kann die Probe von Kapillarplasma aus Kapillarblut auch durch direktes Sammeln des Kapillarbluts in den Probengefäßen erfolgen. Anschließend an die Zentrifugation, bei welcher sich der korpuskuläre Anteil der Blutprobe vom Blutplasma abtrennt, wird dann aus dem Überstand an Blutplasma das überstehende Plasma in gewünschter Menge abpipettiert.

Bei diesem Abpipettieren besteht jedoch in der Regel das Problem, dass dieser Vorgang äußerst sorgfältig zu erfolgen hat, denn es besteht die Gefahr, dass beim Pipettieren der Blutkuchen am Gefäßboden berührt wird und somit Nicht-Plasmabestandteile mitpipettiert werden. Die Plasmafraktion kann dadurch verunreinigt werden, wodurch Messwerte erheblich beeinflusst werden können.

Verringern lässt sich diese Gefahr einer Verunreinigung der Plasmafraktion lediglich durch die Gewinnung unnötig großer Blutmengen bzw. Plasmamengen. Beispielsweise muss in der Regel die ca. 5- bis 7-fache Menge an benötigtem Plasma als Kapillarblut gesammelt werden, um ohne Gefahr einer Verunreinigung die notwendige Plasmamenge abnehmen zu können.

Diese Entnahme größerer Blutmengen bereitet jedoch ihrerseits erhebliche Schwierigkeiten. So sind beispielsweise für übliche Tests, beispielsweise zum quantitativen Nachweis eines oder mehrerer der oben genannten Analyten, typischerweise Mengen von ca. 31 ± 1,5 µl an sauberem Blutplasma erforderlich. Nach den oben genannten Verfahren setzt dies jedoch eine hohe Volumenverfügbarkeit einer Ausgangsmenge an Kapillarblut voraus, welche nicht immer gegeben ist. Beispielsweise wären nach den obigen Angaben zu diesem Zweck ca. 150 - 200 µl Kapillarblut erforderlich, was jedoch in der Praxis oft nur schwer realisierbar ist.

### Aufgabe der Erfindung

Es ist daher eine Aufgabe der vorliegenden Erfindung, Verfahren und Vorrichtungen bereitzustellen, welche die Nachteile bekannter Verfahren und Vorrichtungen zur Bereitstellung eines definierten Volumens eines Zielbestandteils einer Probe zumindest weitgehend vermeiden. Insbesondere sollen die vorgeschlagenen Verfahren und Vorrichtungen eine konstante und präzise Bereitstellung eines Volumens eines Zielbestandteils einer Probe, insbesondere eines Plasmabestandteils einer Blutprobe, bei gleichzeitig hohem Reinheitsgrad und ohne die Gefahr einer Verunreinigung des Zielbestandteils durch andere Bestandteile der Probe ermöglichen.

### Offenbarung der Erfindung

Diese Aufgabe wird durch die Verfahren und Vorrichtungen mit den Merkmalen der unabhängigen Ansprüche gelöst. Vorteilhafte Weiterbildungen der Erfindung, welche einzeln oder in Kombination realisierbar sind, sind in den abhängigen Ansprüchen dargestellt, dabei kann das im Folgenden beschriebene Verfahren insbesondere unter Verwendung einer oder mehrerer der beschriebenen Vorrichtungen durchgeführt werden, und die Vorrichtungen können eingerichtet sein, um ein erfindungsgemäßes Verfahren in einer oder mehreren der beschriebenen Varianten durchzuführen. Dementsprechend kann für mögliche Ausgestaltungen des Verfahrens auf die Beschreibung der Vorrichtungen verwiesen werden und umgekehrt.

Die Erfindung beruht auf der Erkenntnis, dass die aus dem Stand der Technik bekannten Verfahren, bei welchen zunächst mittels einer Kapillare Kapillarblut entnommen wird, dann die Kapillare zur Erzeugung eines definierten Volumens an Blut gebrochen wird und anschließend dieses Blut zentrifugiert wird, sich erheblich dadurch vereinfachen und verbessern lassen, dass das Blut vor dem Zentrifugieren nicht der Kapillare entnommen wird. Die Erfindung betrifft dementsprechend ein Verfahren und Vorrichtungen, welche insbesondere zur Gewinnung und Applikation genau definierter Volumina von Blutplasma eingesetzt werden können. Es kann eine speziell adaptierte Dosierkapillare verwendet werden. Dementsprechend kann die Erfindung eine Kombination einer Blutgewinnung in einer speziellen Kapillare, einer Plasmagewinnung mittels Zentrifugation des Bluts innerhalb dieser Kapillare und schließlich einer volumendosierten Plasma-Applikation durch Abbrechen eines Endes dieser Kapillare mit genau definiertem Innenvolumen und Entleeren dieses definierten Plasmavolumens für nachfolgende Tests beinhalten, beispielsweise durch Auftragen auf einen Teststreifen. Die Erfindung minimiert die benötigte Blutmenge erheblich und macht den manuellen Abtrenn- und Pipettierschritt überflüssig. Gleichzeitig ist die gewonnene Plasmamenge konstant und präzise im Volumen.

Allgemein wird ein Verfahren zur Bereitstellung mindestens eines definierten Volumens eines Zielbestandteils einer Probe vorgeschlagen. Wie oben ausgeführt, kann es sich bei dieser Probe insbesondere um eine Blutprobe, beispielsweise Kapillarblut, und/oder eine andere Probe einer Körperflüssigkeit handeln. Auch andere Proben sind jedoch grundsätzlich einsetzbar.
Unter einem Zielbestandteil kann dabei allgemein ein Bestandteil der Probe verstanden werden, welcher für die Bereitstellung von Interesse ist, beispielsweise um diesen einer weiteren Verwendung zuzuführen. Der Zielbestandteil kann insbesondere, wie oben dargestellt, Blutplasma sein. Unter einer Bereitstellung werden dabei eine Gewinnung dieses Zielbestandteils und/oder die Applikation dieses Zielbestandteils verstanden, insbesondere die Applikation in mindestens einer medizinischen und/oder diagnostischen Verwendung. Unter einem "definierten" Volumen wird ein Volumen verstanden, welches im Rahmen vorgegebener Fehlergrenzen reproduzierbar bereitstellbar ist, beispielsweise im Rahmen von Fehlergrenzen von nicht mehr als ± 5%, vorzugsweise nicht mehr als ± 1%.

Das vorgeschlagene Verfahren umfasst die nachfolgenden Verfahrensschritte, welche vorzugsweise, jedoch nicht notwendigerweise, in der dargestellten Reihenfolge durchgeführt werden. Weiterhin können zusätzliche, nicht dargestellte Verfahrensschritte durchgeführt werden. Zudem können einzelne oder mehrere Verfahrensschritte wiederholt oder zeitlich parallel oder zeitlich überlappend durchgeführt werden.

In einem ersten Verfahrensschritt wird mindestens eine Dosierkapillare bereitgestellt, welche mindestens zwei Öffnungen aufweist. Unter einer Dosierkapillare wird dabei eine Kapillare mit einem Kapillarkanal verstanden, dessen Dimensionierung im Wesentlichen bekannt ist. Zumindest sollte die Dimensionierung so weit bekannt bzw. reproduzierbar sein, dass die oben beschriebenen Toleranzen eingehalten werden können. Die Dosierkapillare bzw. der Dosierkanal können dabei vorzugsweise im Wesentlichen gerade ausgestaltet sein. Es sind jedoch auch andere Geometrien grundsätzlich möglich, beispielsweise eine gekrümmte Dosierkapillare, wie zum Beispiel eine U-förmig gebogene Dosierkapillare.

Die Dosierkapillare weist mindestens zwei Öffnungen auf. Beispielsweise kann die Dosierkapillare zwei einander gegenüberliegende Öffnungen aufweisen, beispielsweise jeweils am gegenüberliegenden Ende des mindestens einen Kapillarkanals der Dosierkapillare. Die Dosierkapillare ist also vorzugsweise als beidseitig geöffnete, vorzugsweise lineare, Dosierkapillare ausgestaltet. Diese mindestens zwei Öffnungen können beispielsweise, wie im Folgenden näher ausgeführt wird, mindestens eine distale Öffnung und mindestens eine proximale Öffnung umfassen. Unter einer proximalen Öffnung wird dabei eine Öffnung verstanden, durch welche eine Befüllung erfolgt, wohingegen unter einer distalen Öffnung eine andere Öffnung verstanden wird, durch welche keine Befüllung erfolgt.

In einem weiteren Verfahrensschritt wird die Dosierkapillare zumindest teilweise mit der Probe befüllt. Die Befüllung erfolgt dabei beispielsweise durch eine oder mehrere der mindestens zwei Öffnungen, also die proximale Öffnung. Die Befüllung erfolgt vorzugsweise zumindest im Wesentlichen vollständig, so dass der Kapillarkanal vorzugsweise vollständig mit der Probe angefüllt ist. Zu diesem Zweck kann eine geringe Menge der Probe aus einer nicht zur Befüllung verwendeten Öffnung, beispielsweise der oben genannten distalen Öffnung, austreten. Überschüssige Anteile, welche aus der distalen Öffnung ausgetreten sind, können nach dem Befüllen entfernt werden, beispielsweise durch einfaches Abwischen. Grundsätzlich kann jedoch auch eine nicht vollständige Befüllung der Dosierkapillare erfolgen, sofern die relevanten Teile des Kapillarkanals, welche später das definierte Volumen bereitstellen, im Wesentlichen vollständig befüllt sind.

Weiterhin umfasst das Verfahren die Durchführung eines Verfahrensschritts, in welchem eine Bestandteilsseparation zur zumindest teilweisen Trennung mindestens zweier Bestandteile der Probe innerhalb der Dosierkapillare erfolgt. Insbesondere können diese mindestens zwei Bestandteile bei einer Blutprobe das bereits genannte Blutplasma sowie korpuskuläre Anteile der Blutprobe (Blutkuchen) umfassen. Alternativ ist jedoch auch eine andere Art von Trennung, beispielsweise eine Trennung in mehr als zwei Bestandteile, möglich. Die Bestandteilsseparation erfolgt dabei, im Unterschied zu den aus dem Stand der Technik bekannten Verfahren, innerhalb der Kapillare selbst, ohne dass die Probe aus der Dosierkapillare entfernt wird. Damit unterscheidet sich das vorgeschlagene Verfahren beispielsweise von den bekannten Verfahren, bei welchen ein abgebrochener Teil einer Kapillare in ein Zentrifugiergefäß eingegeben wird, um dort seinen Inhalt an das Zentrifugiergefäß abzugeben, um anschließend einer Zentrifugation unterworfen zu werden.

Die Bestandteilsseparation kann insbesondere durch Einwirken von Kräften auf die Dosierkapillare und/oder auf die in der Dosierkapillare enthaltene Probe erfolgen. Insbesondere kann es sich dabei um Gewichtskräfte und/oder Scheinkräfte handeln. Gewichtskräfte können beispielsweise im Rahmen einer statischen Separation oder Sedimentation für die Bestandteilsseparation eingesetzt werden. Die Scheinkräfte können beispielsweise eine Zentrifugalkraft umfassen, welche beispielsweise mittels einer Zentrifuge, insbesondere einer Hämatokrit-Zentrifuge oder ähnlichem auf die Dosierkapillare und/oder die Probe innerhalb der Dosierkapillare ausgeübt werden.

In einem weiteren Verfahrensschritt wird die Dosierkapillare in mindestens zwei Teilstücke unterteilt, wobei mindestens eines der Teilstücke das definierte Volumen des Zielbestandteils enthält.

Diese Aufteilung der Dosierkapillare in die mindestens zwei Teilstücke kann auf verschiedene Weisen erfolgen, welche vorzugsweise der Bestandteilsseparation angepasst sind. So können beispielsweise zwei Teilstücke vorgesehen sein, so dass beispielsweise die Dosierkapillare in genau zwei Teilstücke unterteilt werden kann, welche jeweils den Enden der Dosierkapillare entsprechen. Eines dieser Teilstücke kann dann beispielsweise als Ziel-Teilstück verwendet werden und kann das definierte Volumen des Zielbestandteils enthalten. Alternativ ist jedoch auch eine Unterteilung in mehrere Teilstücke möglich, so dass beispielsweise zwei Enden der Dosierkapillare abgebrochen werden und lediglich ein mittleres Teilstück als Ziel-Teilstück verwendet wird, welches das definierte Volumen des Zielbestandteils enthält. Verschiedene Ausgestaltungen sind möglich.

Wie oben dargestellt, kann das Verfahren weiterhin ein Bereitstellen des definierten Volumens des Zielbestandteils an mindestens eine medizinische und/oder diagnostische Verwendung beinhalten. Dieses Bereitstellen kann beispielsweise ein Bereitstellen an ein Analyseverfahren zum Nachweis mindestens eines Analyten in dem Zielbestandteil und/oder ein anderes Verfahren zur Bestimmung mindestens einer anderen Eigenschaft des Zielbestandteils beinhalten. Zum Bereitstellen kann das definierte Volumen des Zielbestandteils beispielsweise aus dem Ziel-Teilstück ausgegeben werden. Hierbei können wiederum beispielsweise Kapillarkräfte verwendet werden, beispielsweise indem eine Teilstücköffnung mindestens eines der Teilstücke, nämlich des Ziel-Teilstücks mit dem darin aufgenommenen definierten Volumen des Zielbestandteils, an ein Testelement und/oder einen Probenträger angenähert wird. Dieses Annähern kann beispielsweise durch Aufsetzen der Teilstücköffnung auf den Probenträger erfolgen. Das Testelement und/oder der Probenträger können beispielsweise eben ausgestaltet sein, beispielsweise als Teststreifen oder ebener Mikroskopträger. Die Teilstücköffnung, aus welcher die Ausgabe, d.h. das Bereitstellen des definierten Volumens des Zielbestandteils, erfolgt, kann beispielsweise eine bereits zuvor in der Dosierkapillare vorhandene Öffnung umfassen, vorzugsweise die distale Öffnung oder die proximale Öffnung. Alternativ kann die Teilstücköffnung jedoch auch mindestens eine Öffnung umfassen, welche erst beim Aufteilen der Dosierkapillare in die mindestens zwei Teilstücke geschaffen wird, beispielsweise eine Öffnung an einer Bruchkante. Bevorzugt ist jedoch die Ausgabe durch eine zuvor bereits vorhandene Öffnung, da diese Öffnung auch bei unterschiedlichen Aufteilungsvorgängen stets definiert verbleibt.

Bei der Bestandteilsseparation können mittels Einwirkung von Zentrifugalkräften und/oder Gravitationskräften insbesondere im Fall einer Blutprobe korpuskuläre Bestandteile der Blutprobe zumindest teilweise von Blutplasma getrennt werden. Das Aufteilen der Dosierkapillare erfolgt dann vorzugsweise derart, dass das definierte Volumen des Zielbestandteils möglichst ausschließlich Blutplasma enthält. Verunreinigungen durch andere Blutbestandteile innerhalb vorgegebener Toleranzgrenzen können dabei jedoch gegebenenfalls in Kauf genommen werden. Alternativ oder zusätzlich können natürlich auch andere Zielbestandteile ausgewählt werden. Beispielsweise können gezielt korpuskuläre Bestandteile als Zielbestandteil gewählt werden. Im Folgenden wird jedoch, ohne Beschränkung weiterer möglicher Ausführungen, von der Auswahl eines Zielbestandteils aus Blutplasma ausgegangen.

Das Auswählen des mindestens einen Zielbestandteils erfolgt, wie oben dargestellt, dadurch, dass die Dosierkapillare gezielt geteilt wird, um den Zielbestandteil nach der Bestandteilsseparation aus der Dosierkapillare auszuwählen. Dies kann insbesondere durch Trennen der Dosierkapillare in mindestens zwei Teilstücke erfolgen. Wie unten exemplarisch näher ausgeführt wird, kann zur Aufteilung der Dosierkapillare beispielsweise mindestens ein mechanisches Bruchverfahren verwendet werden. Beispielsweise kann die Dosierkapillare mindestens eine Sollbruchstelle, beispielsweise in Form einer vollständig oder teilweise umlaufenden Ritzung, aufweisen. Der Begriff einer Ritzung ist dabei weit zu fassen und umfasst grundsätzlich eine beliebige lokale Wandstärkeverringerung. So können beispielsweise auch Schliffe umfasst sein. Insbesondere kann die Ritzung derart ausgestaltet sein, dass diese bei einem Bruch zu glatten Bruchstellen führt. Auch mehrere Sollbruchstellen können vorgesehen sein. Die Sollbruchstelle kann beispielsweise eine Ritzung mit einer Ritztiefe aufweisen, welche zwischen 10 µm und 100 µm liegt, insbesondere zwischen 35 µm und 50 µm, bei einer Wandstärke zwischen 150 µm und 300 µm, insbesondere bei 200 µm. Das Verhältnis der Ritztiefe zur Wandstärke kann beispielsweise zwischen 1/4 und 1/6 liegen.

Das Aufteilen der Dosierkapillare, beispielsweise durch entsprechendes Brechen, kann beispielsweise derart erfolgen, dass das Zielvolumen weniger als 50 % des Kapillarvolumens der Dosierkapillare umfasst, vorzugsweise maximal 45 % und besonders bevorzugt 37 %. Beispielsweise kann eine Kapillare mit einem konstanten Kapillardurchmesser verwendet werden. Beispielsweise kann die Dosierkapillare ein Kapillarvolumen aufweisen, welches zwischen 70 µl und 150 µl liegt, vorzugsweise zwischen 80 µl und 90 µl und besonders bevorzugt bei 84 µl. Bei der genannten Aufteilung kann das Volumen des Zielbestandteils beispielsweise 31 µl betragen, wenn das gesamte Kapillarvolumen ca. 84 µl beträgt.

Insbesondere wenn eine Kapillare mit einem konstanten Kapillardurchmesser als Dosierkapillare verwendet wird, kann die Dosierkapillare beispielsweise in zwei oder mehr Teilstücke unterteilt werden, beispielsweise durch das oben beschriebene Bruchverfahren. So kann die Dosierkapillare beispielsweise in einem Verhältnis x geteilt werden. Dieses Verhältnis x entspricht dann beispielsweise der Teillänge der Kapillare in dem Teilstück, welche das definierte Volumen des Zielbestandteils enthält, zur Gesamtlänge der Dosierkapillare und/oder der Gesamtlänge der ursprünglich befüllten Dosierkapillare. Dieses bevorzugte Verhältnis ergibt sich aus typischen in der Praxis auftretenden Hämatokrit-Werten, welche in den meisten Fällen 60 % nicht übersteigen. Auf diese Weise kann, beispielsweise durch ein Verhältnis x von 37 %, beispielsweise durch entsprechende Wahl der Lage der Sollbruchstelle, sichergestellt werden, dass bei der Verwendung von Blutproben die Aufteilung stets innerhalb eines ausschließlich mit Blutplasma gefüllten Bereichs der Dosierkapillare erfolgt. Das definierte Volumen des Zielbestandteils kann dann insbesondere dem kleineren der beiden Teilstücke entnommen werden, also dem Teilstück mit der Länge von weniger als 50 % der Gesamtlänge der Dosierkapillare oder vorzugsweise maximal 45 % und insbesondere 37% der Gesamtlänge der Dosierkapillare.

Nach Durchführung der Bestandteilsseparation und vor Aufteilung der Dosierkapillare können weitere Schritte durchgeführt werden, beispielsweise um weitere Eigenschaften der Probe zu bestimmen. So kann beispielsweise nach dem Durchführen der Bestandteilsseparation und vor der Aufteilung der Dosierkapillare mindestens ein Zwischenanalyseschritt durchgeführt werden. Bei diesem mindestens einen Zwischenanalyseschritt kann beispielsweise aus der zumindest teilweisen Trennung der mindestens zwei Bestandteile der Probe, beispielsweise bei einer Blutprobe des Blutplasmas und der korpuskulären Bestandteile der Blutprobe, innerhalb der Dosierkapillare, auf mindestens eine Eigenschaft der Probe geschlossen werden. Beispielsweise kann auf einen Anteil korpuskulärer Bestandteile der Blutprobe geschlossen werden, insbesondere auf einen Hämatokrit-Wert.

Dieser Zwischenanalyseschritt kann insbesondere auf vergleichsweise einfache Art und Weise durchgeführt werden, beispielsweise durch eine optische Messung und/oder eine optische Betrachtung. Dies kann vollautomatisch oder auch manuell erfolgen. So kann beispielsweise eine Dosierkapillare aus Glas oder einem anderen zumindest teilweise transparenten Werkstoff verwendet werden, so dass die Trennung der Bestandteile innerhalb der Dosierkapillare optisch beobachtet werden kann. Auf diese Weise kann, durch Feststellung der Lage der mindestens einen Trennlinie zwischen den mindestens zwei Bestandteilen, beispielsweise auf die mindestens eine Eigenschaft, beispielsweise den Hämatokrit-Wert, geschlossen werden. Bei konstantem Kapillardurchmesser der Dosierkapillare kann dies beispielsweise die Länge des mit korpuskulären Bestandteilen gefüllten Anteils der Dosierkapillare gemessen werden, beispielsweise mit einem einfachen Lineal oder anderem Maßstab, und diese Länge zur Gesamtlänge der Dosierkapillare bzw. zur gesamten befüllten Länge der Dosierkapillare ins Verhältnis gesetzt werden, um den Hämatokrit-Wert zu errechnen.

Die mindestens eine bereitgestellte Dosierkapillare kann beispielsweise, wie oben ausgeführt, als lineare Dosierkapillare ausgestaltet sein, auch andere Ausgestaltungen sind möglich. Die Dosierkapillare kann beispielsweise ein distales Ende und ein proximales Ende aufweisen, wobei eine distale Öffnung an dem distalen Ende angeordnet ist und eine proximale Öffnung an dem proximalen Ende. Die distale und proximale Öffnung sind also vorzugsweise an einander gegenüberliegenden Enden der Dosierkapillare angeordnet.

Allgemein ist es besonders bevorzugt, wenn mindestens eine der Öffnungen eine Verengung, insbesondere eine Konstriktion, aufweist. Insbesondere kann mindestens eine distale Öffnung mit einer derartigen Verengung ausgestaltet sein. Mindestens eine proximale Öffnung, also eine Öffnung, durch welche eine Befüllung der Dosierkapillare erfolgt, sollte ohne eine derartige Verengung ausgestaltet sein. Konstriktionen, also nach innen ragende, umlaufende Ränder der Kapillare, sind beispielsweise durch entsprechende Ziehverfahren für Kapillare technisch leicht zu erzeugen. Alternativ oder zusätzlich zu einer Verengung an der distalen Öffnung können natürlich auch andere Öffnungen mit derartigen Verengungen versehen werden. Die Verengung kann insbesondere eine ideale Verringerung des Durchmessers der Dosierkapillare auf die Hälfte oder weniger, insbesondere auf ein Viertel oder weniger, bewirken.

Das Befüllen der Dosierkapillare kann also, wie oben dargestellt, insbesondere von der proximalen Öffnung her erfolgen. Dabei kann, wie oben dargestellt, eine Menge der Probe beim Befüllen an der distalen Öffnung austreten. Auf diese Weise ist sichergestellt, dass die Dosierkapillare vollständig befüllt wird. Vor Durchführung der Bestandteilsseparation und/oder zu anderen Zeitpunkten des Verfahrens kann dann beispielsweise die austretende Menge der Probe entfernt werden, beispielsweise durch Abwischen der Dosierkapillare oder durch andere Reinigungsschritte. Wird eine Dosierkapillare mit einer Verengung, insbesondere einer Konstriktion, verwendet, so kann dieses Austreten der Probe am distalen Ende der Dosierkapillare in der Regel verhindert oder zumindest reduziert werden.

Wie oben dargestellt, kann nach Aufteilung der Dosierkapillare und somit nach Gewinnung des definierten Volumens des Zielbestandteils dieses definierte Volumen des Zielbestandteils insbesondere an mindestens eine medizinische und/oder diagnostische Verwendung erfolgen. Dieses Bereitstellen kann beispielsweise durch mindestens eine der Öffnungen erfolgen. So kann beispielsweise eine der Trennlinie zuweisende Öffnung verwendet werden, beispielsweise eine Öffnung an einer Bruchstelle nach Brechen der Dosierkapillare. Da die Bruchkanten jedoch unter Umständen undefiniert sein können, ist es besonders bevorzugt, wenn die Bereitstellung des definierten Volumens des Zielbestandteils an die mindestens eine medizinische und/oder diagnostische Verwendung durch die ursprünglich vorhandene Öffnung, beispielsweise die distale Öffnung, hindurch erfolgt.

Das Befüllen der Dosierkapillare erfolgt, wie oben dargestellt, vorzugsweise derart, dass diese im Wesentlichen vollständig durch die Probe gefüllt wird. Dies kann beispielsweise durch das genannte Austreten einer geringfügigen Menge der Probe aus der distalen Öffnung erfolgen. Vor Durchführung der Bestandteilsseparation, insbesondere durch ein Zentrifugierverfahren, sollte mindestens eine der Öffnungen verschlossen werden. Insbesondere kann die proximale Öffnung verschlossen werden. Alternativ oder zusätzlich kann jedoch auch eine andere Öffnung, beispielsweise jeweils diejenige Öffnung, zu welcher hin die Probe bei der Bestandteilsseparation gedrückt wird, verschlossen werden. Dies kann wiederum insbesondere die proximale Öffnung sein. Auch andere Ausgestaltungen sind jedoch grundsätzlich möglich.

Das Verschließen der mindestens einen Öffnung kann auf verschiedene Weisen erfolgen. Insbesondere können eine oder mehrere der folgenden Verschlüsse verwendet werden: ein Kitt; eine Kappe, insbesondere eine Kunststoff-Kappe, vorzugsweise eine Silikonkappe; ein Wachs, insbesondere ein Hämatokrit-Wachs; ein Harz; ein Klebstoff.

Für die mindestens eine Dosierkapillare können grundsätzlich aus dem Stand der Technik bekannte Materialien verwendet werden. Beispielsweise kann die mindestens eine Dosierkapillare mindestens einen Glaswerkstoff umfassen und/oder vollständig aus Glas hergestellt sein. Auch andere Werkstoffe sind jedoch grundsätzlich möglich, beispielsweise Quarze, Keramiken, Kunststoffe oder ähnliches. Insbesondere kann der verwendete Werkstoff auf das jeweils verwendete Trennverfahren angepasst sein. Werden Bruchverfahren verwendet, so werden vorzugsweise harte, spröde Werkstoffe verwendet. Werden andere Trennverfahren verwendet, wie beispielsweise Schneideverfahren, so werden vorzugsweise leicht schneidbare Werkstoffe verwendet, wie beispielsweise Kunststoffe. Insbesondere lassen sich transparente oder zumindest teilweise transparente Werkstoffe verwenden.

Die Dosierkapillare kann insbesondere einen Kapillarinnendurchmesser zwischen 0,5 mm und 4 mm, insbesondere zwischen 1,0 und 1,2 mm, aufweisen. Diese Kapillardurchmesser haben sich in der Praxis zum Aufnehmen insbesondere einer Blutprobe als geeignet erwiesen. Auch andere Kapillardurchmesser sind jedoch grundsätzlich möglich.

Die Dosierkapillare kann weiterhin auch einen oder mehrere Wirkstoffe umfassen. Insbesondere kann die Dosierkapillare mindestens einen Antikoagulanz-Wirkstoff aufweisen, also einen Wirkstoff, welcher eine Koagulation einer Blutprobe zumindest teilweise verhindert. Dieser Wirkstoff kann beispielsweise in das Material der Dosierkapillare eingebracht sein. Besonders bevorzugt ist es jedoch, wenn alternativ oder zusätzlich, der Wirkstoff als Beschichtung auf die Innenseite der Dosierkapillare aufgebracht wird, insbesondere in Form einer Antikoagulanz-Beschichtung. Hierbei können gängige Antikoagulanzien verwendet werden, beispielsweise eine EDTA-Beschichtung (Ethylendiamintetraacetat). Auch andere Antikoagulanzien sind jedoch bekannt und können alternativ oder zusätzlich eingesetzt werden.

Neben dem beschriebenen Verfahren in einer der beschriebenen Verfahrensvarianten wird weiterhin eine Dosierkapillare vorgeschlagen, welche insbesondere zur Verwendung in einem Verfahren nach einem der vorhergehenden Ansprüche geeignet sein kann. Diese Dosierkapillare umfasst mindestens zwei Öffnungen. Mindestens eine der Öffnungen weist mindestens eine Verengung auf, insbesondere mindestens eine Konstriktion. Die Dosierkapillare weist weiterhin zwischen den Öffnungen mindestens eine Trennlinie, insbesondere mindestens eine Trennlinie mit mindestens einer Sollbruchstelle, auf Unter einer Trennlinie kann dabei allgemein eine Linie verstanden werden, insbesondere eine senkrecht zu einer Längserstreckung der Kapillare verlaufende Linie, entlang welcher, insbesondere für einen Benutzer optisch erkennbar, eine Trennung vorgenommen werden kann. Alternativ oder zusätzlich zu der Sollbruchstelle kann die Trennlinie auch auf andere Weise ausgestaltet sein, beispielsweise mit mindestens einer Trennmarkierung, beispielsweise in Form einer Ringmarkierung oder ähnlichem, welche einen Ort einer Trennung der Dosierkapillare bei der Aufteilung der Dosierkapillare in die mindestens zwei Teilstücke kennzeichnet. Für weitere mögliche Einzelheiten kann auf die obige Beschreibung verwiesen werden.

Neben dem Verfahren und der Dosierkapillare in einer oder mehreren der oben beschriebenen Ausführungsvarianten wird weiterhin eine Vorrichtung zum Bereitstellen mindestens eines definierten Volumens eines Zielbestandsteils einer Probe, insbesondere einer Blutprobe, vorgeschlagen, insbesondere zur Bereitstellung eines definierten Volumens an Blutplasma. Die Vorrichtung kann insbesondere eingerichtet sein, um ein Verfahren gemäß einer oder mehreren der oben beschriebenen Ausführungsvarianten durchzuführen. Die Vorrichtung umfasst mindestens eine Dosierkapillare, insbesondere eine Dosierkapillare der zuvor beschriebenen Art. Die Dosierkapillare umfasst mindestens zwei Öffnungen und mindestens eine Trennlinie, insbesondere eine Trennlinie mit mindestens einer Sollbruchstelle und/oder mindestens einer Trennmarkierung. Weiterhin umfasst die Vorrichtung mindestens eine Separationsvorrichtung zum Durchführen einer Bestandteilsseparation zur zumindest teilweisen Trennung mindestens zweier Bestandteile der Blutprobe innerhalb der Dosierkapillare. Diese Separationsvorrichtung kann insbesondere eine Zentrifuge umfassen, beispielsweise eine Hämatokrit-Zentrifuge. Für weitere optionale Ausgestaltungen der Vorrichtung kann beispielsweise auf die obige Beschreibung verwiesen werden.

Die Vorrichtung kann weiterhin mindestens eine Halterungsvorrichtung umfassen, welche eingerichtet ist, um die Dosierkapillare in einer definierten Position für ein zumindest teilweises Befüllen der Dosierkapillare mit der Probe zu fixieren. Insbesondere kann es sich bei dieser definierten Position, wobei sowohl räumliche Positionen als auch Ausrichtungen unter diesem Begriff subsumiert werden können, um eine im Wesentlichen waagerechte Position handeln. Unter einer im Wesentlichen waagerechten Position ist dabei eine Position zu verstehen, bei welcher die Dosierkapillare mit der Horizontalen einen Winkel von 0° einnimmt, wobei jedoch auch leichte Abweichungen toleriert werden können, beispielsweise Abweichungen um nicht mehr als 20°, vorzugsweise um nicht mehr als 5°. Auch andere Ausrichtungen sind jedoch grundsätzlich möglich. Die Halterungsvorrichtung kann beispielsweise einen Kapillarhalter umfassen, beispielsweise in Form einer einfachen Kapillarklemme. Alternativ oder zusätzlich kann die Halterungsvorrichtung beispielsweise auch Bestandteil der Separationsvorrichtung zum Durchführen der Bestandteilsseparation sein, so dass beispielsweise in der Halterungsvorrichtung auch anschließend die oben beschriebene Bestandteilsseparation durchgeführt werden kann.

Das vorgeschlagene Verfahren, die vorgeschlagene Dosierkapillare und die vorgeschlagene Vorrichtung weisen gegenüber bekannten Verfahren und Vorrichtungen dieser Art zahlreiche Vorteile auf. So erlaubt das vorgeschlagene Verfahren beispielsweise die Gewinnung einer exakt definierten Probe an Kapillarplasma, beispielsweise genau 31 µl aus 84 µl Kapillarblut, auf sehr einfache Weise und unabhängig vom aktuellen Hämatokrit-Wert. Insbesondere die Dosierkapillare mit der mindestens einen Verengung, insbesondere der Konstriktion, vorzugsweise am distalen Ende, erweist sich als vorteilhaft. Die Konstriktion kann insbesondere einerseits ein Auslaufen des Plasmas beim und nach dem Trennen der Dosierkapillare, beispielsweise dem Abbrechen der Dosierkapillare, und andererseits auch das Überfüllen der Dosierkapillare beim Befüllvorgang durch Abbruch des Kapillartransports vermeiden. Grundsätzlich erweist sich eine derartige Verengung, beispielsweise auch bei einer schrägen Lagerung der Dosierkapillaren, als vorteilhaft.

Die Befüllung der Dosierkapillare ist grundsätzlich von einer oder mehreren Öffnungen aus möglich. Das Verschließen der Dosierkapillaren kann beispielsweise am proximalen Ende erfolgen. Alternativ ist auch ein Verschließen des distalen Endes grundsätzlich möglich. In diesem Fall ergibt sich der Vorteil, dass der Verschluss, beispielsweise ein Kitt-Block, zum Verschließen des distalen Endes der Dosierkapillare weniger mit der Probe kontaminiert wird. So kann das distale Ende mit dem Kitt verschlossen werden, welches nicht an seiner Außenseite in Kontakt mit der Blutabnahmestelle, beispielsweise auf einem Finger, war.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktion einander entsprechende Elemente.

### Im Einzelnen zeigt:

- Figur 1: ein Ausführungsbeispiel einer erfindungsgemäßen Dosierkapillare; und
- Figuren 2A bis 2F: Verfahrensschritte eines Ausführungsbeispiels eines erfindungsgemäßen Verfahrens.

### Ausführungsbeispiele

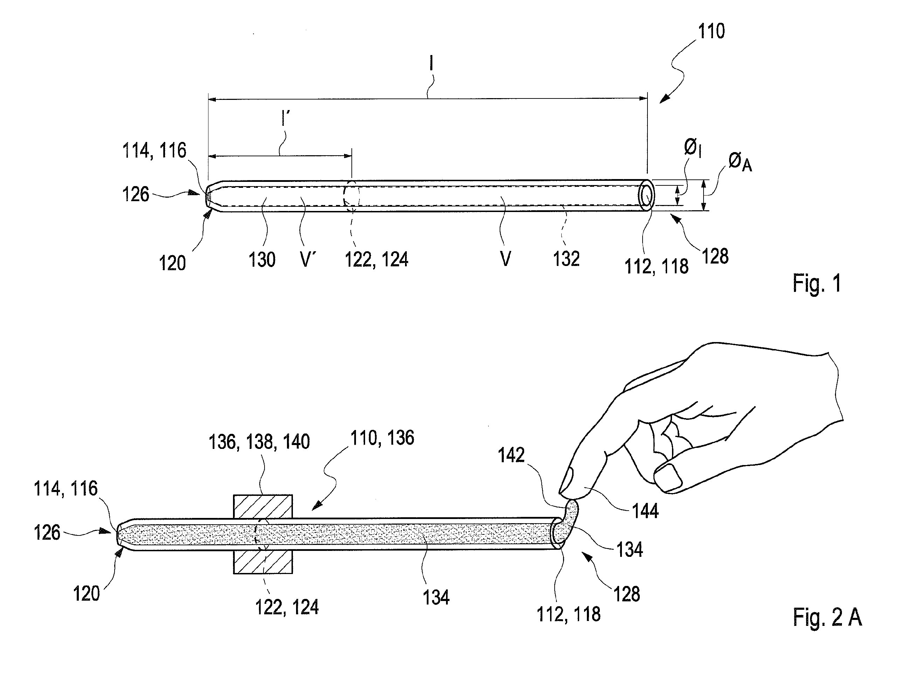

In Figur 1 ist ein einfaches Ausführungsbeispiel einer erfindungsgemäßen Dosierkapillare 110 dargestellt. Die Dosierkapillare 110 ist beispielsweise als Glaskapillare ausgestaltet und weist beispielsweise eine Wandstärke zwischen 0,05 und 5 mm, insbesondere bei ca. 0,2 mm, auf. Die Dosierkapillare weist eine Länge 1 von vorzugsweise zwischen 50 und 150 mm, insbesondere von ca. 75 mm, auf. Der Innendurchmesser der Dosierkapillare liegt beispielsweise zwischen 1,1 und 1,2 mm, wohingegen der Außendurchmesser beispielsweise 1,6 mm betragen kann. Der Außendurchmesser ist dabei in Figur 1 mit ∅A bezeichnet, wohingegen der Innendurchmesser mit ∅_{I} bezeichnet ist.

Die Dosierkapillare weist in dem dargestellten Ausführungsbeispiel vorzugsweise einen im Wesentlichen konstanten Innendurchmesser ∅_{I} auf, so dass sich das Innenvolumen der Dosierkapillare 110 gleichmäßig über die Länge der Dosierkapillare 110 verteilt.

Die Dosierkapillare 110 ist im dargestellten Ausführungsbeispiel als gerade, beidseitig geöffnete Dosierkapillare 110 ausgestaltet, mit zwei einander gegenüberliegenden Öffnungen 112, 114. Die erste dieser beiden Öffnungen 112, 114 wird dabei im Folgenden als distale Öffnung 116 bezeichnet, wohingegen die zweite dieser Öffnungen 112, 114 als proximale Öffnung 118 bezeichnet wird. Wie aus Figur 1 ersichtlich ist, weist im dargestellten Ausführungsbeispiel die distale Öffnung 116 eine Verengung in Form einer Konstriktion 120 auf. Bei dieser Konstriktion 120 ist der Innendurchmesser ∅_{I} der Dosierkapillare 110 beispielsweise auf die Hälfte, insbesondere auf ein Viertel oder weniger, reduziert.

Weiterhin weist die Dosierkapillare 110 eine Trennlinie 122 auf. Diese Trennlinie 122 kann beispielsweise eine optisch wahrnehmbare Markierung umfassen. Besonders bevorzugt umfasst diese Trennlinie 122 jedoch, wie in Figur 1 dargestellt, eine Sollbruchstelle 124, bei welcher die Dosierkapillare 110 eingeritzt ist. Die Ritztiefe dieser Ritzung kann beispielsweise 1/20 bis ½, insbesondere 1/4 bis 1/6 der Wandstärke der Dosierkapillare 110 betragen. Beispielsweise ist bei einer Wandstärke von ca. 0,2 mm eine Ritztiefe, beispielsweise eine Nuttiefe, von 35 µm bis 50 µm bevorzugt.

Die Trennlinie 122 ist in einem Abstand 1' von einer der beiden Öffnungen 112, 114 angeordnet, vorzugsweise von der distalen Öffnung 116. Das Verhältnis 1^{'} zu 1 wird im Folgenden auch mit x bezeichnet. Vorzugsweise ist x < 50 % und beträgt insbesondere maximal 45 % oder weniger, insbesondere 37%. Dementsprechend ist im Inneren der Dosierkapillare 110 zwischen einem distalen Ende 126, an welchem sich die distale Öffnung 116 befindet und der Trennlinie 122 ein Volumen V' aufgenommen, welches sich zum Gesamtvolumen V zwischen dem distalen Ende 126 und einem proximalen Ende 128 wie x verhält. Beispielsweise kann das Gesamtvolumen V ca. 84 µl umfassen, wohingegen das definierte Volumen V^{'}, welches im Folgenden auch mit der Bezugsziffer 130 bezeichnet wird, vorzugsweise bei 31 µl ± 1,5 µl liegt. Auch andere Volumina bzw. Aufteilungen sind jedoch grundsätzlich möglich. Das definierte Volumen 130 nimmt im nachfolgend beschriebenen Verfahren den Zielbestandteil auf.

Die Dosierkapillare 110 kann optional auf verschiedene Weisen weitergebildet werden. So kann die Dosierkapillare 110 beispielsweise wie in Figur 1 ebenfalls angedeutet, auf ihrer Innenseite eine Antikoagulanz-Beschichtung 132 aufweisen, beispielsweise eine EDTA-Beschichtung.

In den Figuren 2A bis 2F sind Verfahrensschritte eines Ausführungsbeispiels eines erfindungsgemäßen Verfahrens zum Bereitstellen eines definierten Volumens eines Zielbestandteils einer Probe 134 dargestellt. Als Probe 134 dient dabei im vorliegenden Beispiel eine Blutprobe, welche beispielsweise in Blutplasma und korpuskuläre Bestandteile zerlegt wird. Zwischen der Probe 134 vor und nach dem Verfahren wird dabei im Folgenden begrifflich nicht unterschieden, so dass diese einheitlich mit der Bezugsziffer 134 bezeichnet wird. Gleichzeitig zeigen diese Figuren die Verwendung einer Dosierkapillare 110 sowie ausschnittsweise einer erfindungsgemäßen Vorrichtung 136 zur Bereitstellung eines definierten Volumens eines Zielbestandteils der Probe 134.

In Figur 2A wird zunächst eine Dosierkapillare 110 bereitgestellt, welche Bestandteil der Vorrichtung 136 ist. Beispielsweise kann es sich dabei um eine Dosierkapillare 110 gemäß der in Figur 1 beschriebenen Art handeln. Beispielsweise kann eine ca. 84 µl fassende, mitfassende, mit EDTA beschichtete Dosierkapillare 110 mit einer Länge 1 von 75 mm und mit einem Innendurchmesser ∅_{I} von 1,2 mm verwendet werden, die an ihrem distalen Ende 126 eine leichte Konstriktion aufweist. Weiterhin kann die Dosierkapillare 110 an einer geeigneten Stelle, beispielsweise in einer Entfernung 1' vom distalen Ende 126 von ca. 27 mm, eine Trennlinie 122, insbesondere eine Sollbruchstelle 124, aufweisen.

In Figur 2A ist weiterhin ein Vorgang dargestellt, bei welchem die Dosierkapillare 110 von einer ihrer Öffnungen 112, 114 hier mit der Probe 134, beispielsweise in Form von Kapillarblut, befüllt wird. In dem dargestellten Ausführungsbeispiel erfolgt dieses Befüllen von der Öffnung 112 her, die dadurch definitionsgemäß zur proximalen Öffnung 118 wird. Alternativ könnte jedoch auch eine Befüllung von der anderen Öffnung 114 her erfolgen. Die Befüllung erfolgt vorzugsweise zumindest näherungsweise vollständig.

Während des Befüllens kann die Dosierkapillare 110 beispielsweise in einer Halterungsvorrichtung 138 aufgenommen sein. Diese Halterungsvorrichtung 138 kann beispielsweise als Einspannvorrichtung ausgestaltet sein. Diese Halterungsvorrichtung 138 kann auch, wie in Figur 2A durch die Bezugsziffer 140 angedeutet, Bestandteil einer Separationsvorrichtung sein, beispielsweise einer Zentrifuge, insbesondere einer Hämatokrit-Zentrifuge. Auch eine separate Halterungsvorrichtung 138 ist jedoch grundsätzlich denkbar. Die Haltungsvorrichtung 138 kann insbesondere eingerichtet sein, um die Dosierkapillare 110 in waagerechter Position zu halten. Hierdurch kann ein Befüllen der Dosierkapillare 110 aus einem Blutstropfen 142 auf einer Fingerbeere 144 eines Patienten ermöglicht werden. Alternativ oder zusätzlich können jedoch auch andere Arten von Proben bereitgestellt werden, beispielsweise Proben aus einem separaten Behältnis, welches zuvor mit Blut befüllt wird. Die vorgeschlagene Befüllung unmittelbar aus einem Blutstropfen 142 heraus weist jedoch zahlreiche Vorteile auf.

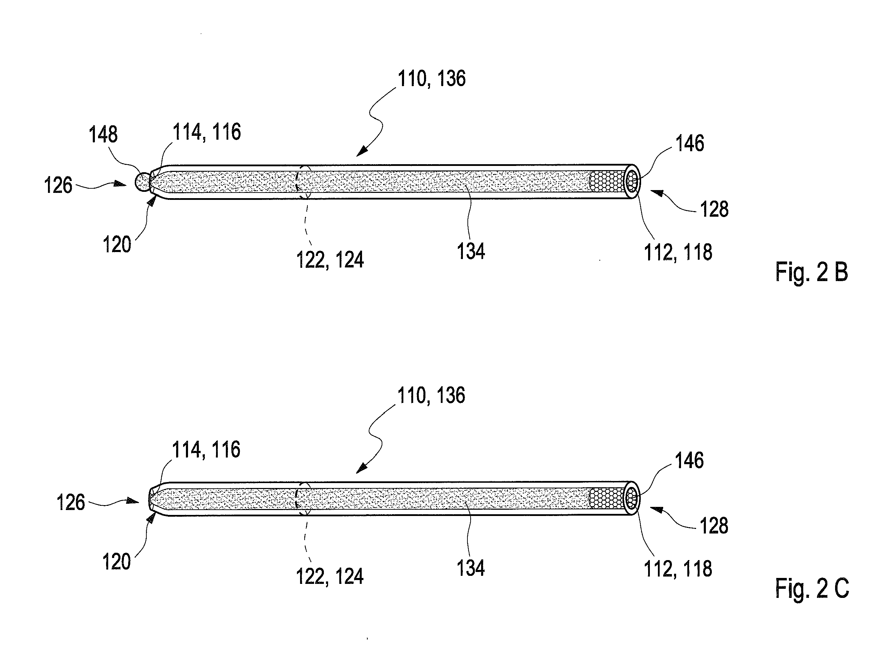

Nach dem in Figur 2A dargestellten Befüllen der Dosierkapillare 110 erfolgt in einem in Figur 2B dargestellten Verfahrensschritt ein Verschließen mindestens einer Öffnung 112, 114 der Dosierkapillare 110. Dabei kann die Dosierkapillare 110 noch, was in Figur 2B nicht dargestellt ist, in der Halterungsvorrichtung 138 aufgenommen sein. Im in Figur 2B dargestellten Ausführungsbeispiel wird dabei die proximale Öffnung 118 verschlossen. Allgemein kann insbesondere diejenige der Öffnungen 112, 114 verschlossen werden, welche sich in einem unten beschriebenen Separationsschritt bei Verwendung einer Zentrifuge als Separationsvorrichtung 140, von der Rotationsachse der Zentrifuge am weitesten entfernt befindet, so dass die Probe 134 zu dieser verschlossenen Öffnung 112, 114 hin gedrückt wird. Im vorliegenden Beispiel ist das die proximale Öffnung 118.

Das Verschließen der Öffnung 112, 114 kann beispielsweise mit einem Verschluss 146 erfolgen, welcher beispielsweise eine Silikonkappe, ein Hämatokrit-Wachs, ein Harz oder einen geeigneten Kleber umfassen kann.

Bereits beim in Figur 2A dargestellten Befüllen der Dosierkapillare 110, jedoch auch beim Verschließen der proximalen Öffnung 118 mit dem Verschluss 146 macht sich insbesondere die Verengung in Form der Konstriktion 120 am gegenüberliegenden, distalen Ende 126 der Dosierkapillare 110 positiv bemerkbar. So kann diese Konstriktion 120 beispielsweise beim Befüllen oder auch beim Aufsetzen des Verschlusses 146 ein Auslaufen der Probe 134 aus der distalen Öffnung 116 im größeren Maßstab verhindern. Wie in Figur 2B dargestellt, kann jedoch eine kleinere Menge der Probe 134 aus der distalen Öffnung 116 austreten und anschließend beispielsweise durch einfaches Abwischen entfernt werden. Dieser Überstand, welcher aus der distalen Öffnung 116 austritt, ist in Figur 2B mit der Bezugsziffer 148 bezeichnet.

In Figur 2C ist schließlich die fertig befüllte, mit dem Verschluss 146 verschlossene Dosierkapillare 110 dargestellt. Diese kann noch in der Halterungsvorrichtung 138 aufgenommen sein, was in Figur 2C wiederum nicht dargestellt ist. Auch eine andere Lagerung ist jedoch grundsätzlich möglich, sowie alternativ oder zusätzlich ein Transport der Dosierkapillare 110. Zu diesem Zweck kann beispielsweise auch die distale Öffnung 116 verschlossen werden.

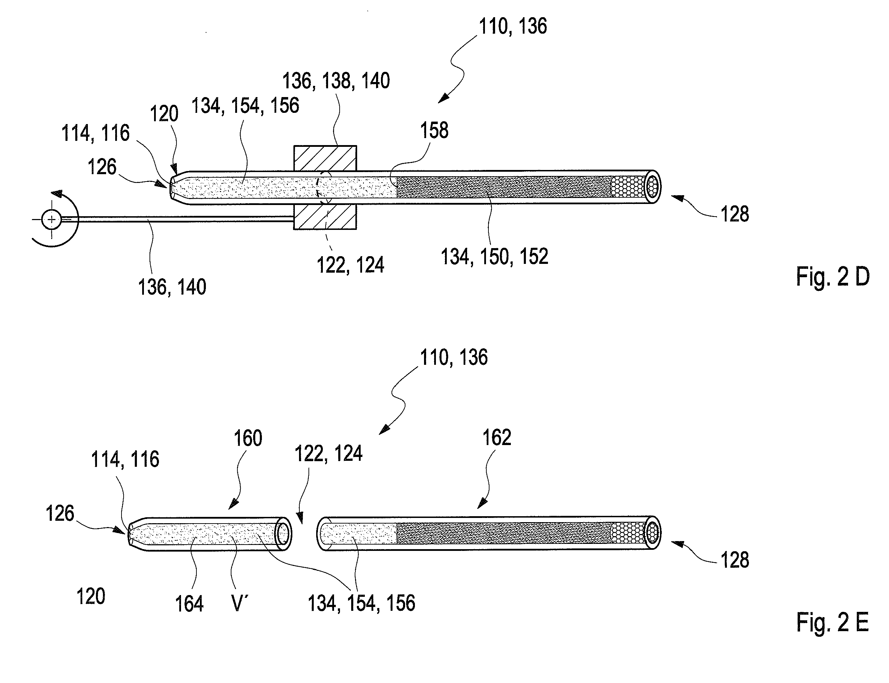

Anschließend erfolgt ein in Figur 2D symbolisch dargestellter Schritt einer Bestandteilsseparation. Bei dieser Bestandteilsseparation erfolgt eine zumindest weitgehende Trennung eines ersten Bestandteils der Probe 134, welcher korpuskuläre Anteile 152 der Probe 134 umfasst, von einem zweiten Bestandteil 154, welcher im dargestellten Beispiel Blutplasma 156 umfasst. Dies erfolgt beispielsweise mittels der oben beschriebenen Separationsvorrichtung 140, insbesondere einer Zentrifuge. Die Zentrifuge kann beispielsweise als Hämatokrit-Zentrifuge oder ähnliches ausgestaltet sein. Insbesondere kann eine einfache Zentrifuge verwendet werden, ohne Einstellmöglichkeiten, beispielsweise mit einer fest vorgegebenen Drehzahl und/oder Laufzeit. Die Trennung der beiden Bestandteile 150, 154 erfolgt in diesem Fall durch Zentrifugalkräfte, wobei die dichteren, korpuskulären Anteile 152 hin zum proximalen Ende 128 der Dosierkapillare 110 getrieben werden. Die leichteren Bestandteile des Blutplasmas 156 lagern sich hingegen zum distalen Ende 126 hin an. Zwischen den beiden Bestandteilen 150, 154, bildet sich, wie in Figur 2D angedeutet, eine Phasengrenze 158 aus. Die Lage dieser Phasengrenze 158 wird durch den aktuellen Hämatokrit-Wert bestimmt. Die Trennlinie 122, beispielsweise die Sollbruchstelle 124, ist derart in ihrer Lage gewählt, dass diese sich bei üblicherweise auftretenden Hämatokrit-Werten innerhalb des Bereichs des zweiten Bestandteils 154 befindet, jedoch möglichst nahe an der Phasengrenze 158. Insbesondere kann diese, wie oben beschrieben, 30 mm vom distalen Ende 126 entfernt angeordnet sein.

Bei dem Separationsschritt können sich gegebenenfalls Luftbläschen in der Dosierkapillare 110 am distalen Ende 126 anlagern. Sollte dies der Fall sein, so können diese beispielsweise in einem weiteren, optionalen Verfahrensschritt zu der distalen Öffnung 116 heraus gedrückt werden, beispielsweise indem an der proximalen Öffnung 118 erneut Kitt des Verschlusses 146 nachgeschoben wird. Beispielsweise kann das proximale Ende 128 nach dem Separationsschritt erneut in eine Kittmatte hineingedrückt werden. So kann sichergestellt werden, dass das definierte Volumen 130 keine Luftbläschen mehr enthält.

Nach dem Separationsschritt kann die Dosierkapillare 110 der Separationsvorrichtung 140 entnommen werden und in mindestens zwei Teilstücke 160, 162 unterteilt werden. Dies ist in Figur 2E durch einfaches Durchbrechen der Dosierkapillare 110 entlang der Sollbruchstelle 124 angedeutet. Alternativ kann jedoch auch ein Unterteilen der Dosierkapillare 110 in mehr als zwei Teilstücke erfolgen.

Durch das Auftrennen der Dosierkapillare 110 in die Teilstücke 160, 162 wird ein Zielbestandteil 164 ausgewählt. In diesem dargestellten Ausführungsbeispiel ist dieser Zielbestandteil 164 ein möglichst großer Anteil des Blutplasmas 156, welches den zweiten Bestandteil 154 der Probe 134 bildet. Der Zielbestandteil 164 weist dabei, wie bereits anhand der Figur 1 beschrieben, das exakt definierte Volumen V' auf.

Das derart gewonnene Blutplasma 156 im Zielbestandteil 164 kann, beispielsweise je nach Beschichtung 132 der Dosierkapillare 110, mit verschiedenen Antikoagulanzien versetzt sein. Es kann jedoch auch Blutplasma 156 aus nicht antikoaguliertem Blut hergestellt werden, beispielsweise indem entsprechend schnell gearbeitet wird.

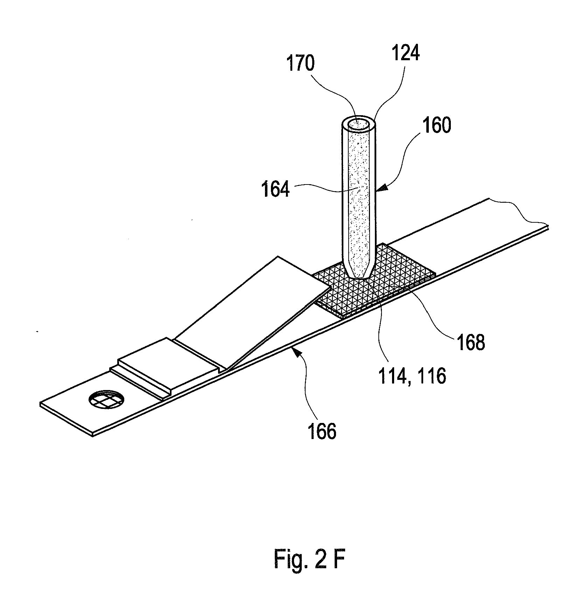

Das abzentrifugierte Zielvolumen des Zielbestandteils 164 kann beispielsweise, wie oben beschrieben, genau 31 µl betragen. Dieser Zielbestandteil 164 kann beispielsweise auf ein Testelement 166 aufgebracht werden. Dies ist exemplarisch in Figur 2F dargestellt. Hierbei ist exemplarisch ein Testelement 166 in Form eines Teststreifens gezeigt, welcher mindestens ein Testfeld 168 aufweist. Beispielsweise kann dieses Testfeld 168 eine entsprechende Testchemie umfassen. Als Beispiel kann ein HDL-P-Reagenzträger für ein Analysegerät vom Typ Reflotron^{®} verwendet werden. Das Aufbringen des Zielbestandteils 164 in dem ersten Teilstück 160 kann beispielsweise dadurch erfolgen, dass die distale Öffnung 116 mit der Konstriktion 120 in Kontakt mit einem Trägernetz des Testfeldes 168 gebracht wird. Alternativ kann jedoch auch eine der Sollbruchstelle 124 zuweisende Öffnung 170, welche beim in Figur 2E dargestellten Trennschritt der beiden Teilstücke 160, 162 entsteht, auf das Trägernetz aufgebracht werden. Bevorzugt ist jedoch die dargestellte Variante, bei welcher eine der bereits ursprünglich vorhandenen Öffnungen 112, 114 vorzugsweise eine Öffnung 114 mit einer Konstriktion 120, auf das Testfeld 168 aufgebracht wird, da diese Öffnung eine definiertere Grenzfläche bereit stellt, beispielsweise eine glatte, geglättete oder polierte Grenzfläche. Anschließend an das Aufbringen auf das Testelement 166 kann beispielsweise eine Messung einer Eigenschaft der Probe 134 erfolgen, welche nunmehr eine Plasmaprobe ist, beispielsweise ein qualitativer und/oder quantitativer Nachweis mindestens eines Analyten in der Plasmaprobe. Beispielsweise kann High-Density-Lipoprotein (HDL) nachgewiesen werden.

Das beschriebene Verfahren und die dargestellte Vorrichtung 136 ermöglichen weiterhin auch eine Bestimmung weiterer Eigenschaften der Kapillarblutprobe. So können auch ein oder mehrere Zwischenanalysen durchgeführt werden, beispielsweise nach dem in Figur 2D dargestellten Separationsschritt, jedoch vor dem in Figur 2E dargestellten Schritt der Trennung der beiden Teilstücke 162, 164. So kann beispielsweise die Lage der Phasengrenze 158 bestimmt werden, beispielsweise mittels einer entsprechenden Messvorrichtung. Auf diese Weise kann beispielsweise unmittelbar auf einen Anteil der korpuskulären Anteile 152 und somit auf den Hämatokrit-Wert der Probe 134 geschlossen werden. Dies kann beispielsweise erfolgen, indem mit Hilfe eines kalibrierten Lineals die Länge der E-rythrozytensäule, also die Länge der korpuskulären Anteile 152 des ersten Bestandteils bestimmt wird. Da die Dosierkapillare 110 stets gleichmäßig und vorzugsweise vollständig gefüllt wird, ist ein Nomogramm, ähnlich dem der Hämatokrit-Bestimmung aus Hämatokrit-Röhrchen, in der Regel nicht erforderlich.

Nach der Messung können beide Teilstücke 160, 162 der Dosierkapillare 110 verworfen werden oder weiteren Messungen zugeführt werden. Die zur Herstellung der Dosierkapillare mit geeigneten Markierungen und/oder Sollbruchstellen 124 erforderlichen Techniken sind dem Fachmann grundsätzlich bekannt. Die erforderliche Genauigkeit für die zu gewinnende Menge an Zielbestandteil 164, beispielsweise Blutplasma, ist fertigungstechnisch kein Problem.

Auch die Separationsvorrichtung 140, zum Beispiel die Zentrifuge, stellt in der Regel keinerlei Ansprüche an die Genauigkeit von Umdrehungszahl und/oder Laufzeit. Die entstehenden Zentrifugalkräfte sollten so niedrig wie möglich gewählt werden, um die Dichtstelle am Verschluss 146 am proximalen Ende 128 der Dosierkapillare 110 nicht übermäßig zu belasten. Beispielsweise können relative Zentrifugalkräfte (relative centrifugal force, rcf) von 5.000 g bis 10.000 g verwendet werden, insbesondere 7.000 bis 9.000 g und besonders bevorzugt 8.000 g, wobei g die Erdbeschleunigung ist. Die vorgeschlagenen Kapillarmaße sind beispielsweise für einen Hämatokrit-Wert bis 60% ausreichend, um 31 µL Blutplasma gewinnen zu können.

Durch geeignete Positionierung der Sollbruchstelle 124 am distalen Ende 126 der Dosierkapillare 110 bzw. durch Änderungen im Innendurchmesser der Dosierkapillaren 110 sind auch andere, definierte Plasmamengen bzw. andere Mengen an Zielbestandteil 164 zu gewinnen. Die Vorgehensweise ändert sich hierdurch nicht.

Es sei darauf hingewiesen, dass die beschriebene Dosierkapillare 110 auch eingesetzt werden kann, ohne dass ein Separationsschritt durchgeführt wird. So kann beispielsweise ohne einen Zentrifugationsschritt mittels der Dosierkapillare 110 eine vorgegebene Blutmenge appliziert werden.

Weiterhin wird darauf hingewiesen, dass das Verfahren in den Figuren 2A - 2F lediglich symbolisch dargestellt ist und beliebig im Rahmen der vorliegenden Erfindung modifiziert werden kann. So kann beispielsweise die Verengung, insbesondere die Konstriktion 120 auch, alternativ oder zusätzlich, an einer anderen Öffnung 112, 114 vorgesehen sein. Weiterhin kann die Befüllung auch von einer anderen Öffnung 112, 114 her erfolgen. Verschiedene Variationen des dargestellten Verfahrens sind denkbar.

### Bezuaszeichenliste

- 110: Dosierkapillare
- 112: Öffnung
- 114: Öffnung
- 116: distale Öffnung
- 118: proximale Öffnung
- 120: Konstriktion
- 122: Trennlinie
- 124: Sollbruchstelle
- 126: distales Ende
- 128: proximales Ende
- 130: definiertes Volumen
- 132: Antikoagulanz-Beschichtung
- 134: Probe
- 136: Vorrichtung zur Bereitstellung eines Zielbestandteils einer Blutprobe
- 138: Halterungsvorrichtung
- 140: Separationsvorrichtung
- 142: Blutstropfen
- 144: Fingerbeere
- 146: Verschluss
- 148: Überstand
- 150: erster Bestandteil
- 152: korpuskuläre Anteile
- 154: zweiter Bestandteil
- 156: Blutplasma
- 158: Phasengrenze
- 160: erstes Teilstück
- 162: zweites Teilstück
- 164: Zielbestandteil
- 166: Testelement
- 168: Testfeld
- 170: Öffnung

## Patentansprüche

1. Verfahren zur Bereitstellung mindestens eines definierten Volumens eines Zielbestandteils (164) einer Probe (134), umfassend die folgenden Schritte:
- Bereitstellen mindestens einer Dosierkapillare (110) mit mindestens zwei Öffnungen (112, 114);
- zumindest teilweises Befüllen der Dosierkapillare (110) mit der Probe (134);
- Durchführen einer Bestandteilsseparation zur zumindest teilweisen Trennung mindestens zweier Bestandteile (150, 154) der Probe (134) innerhalb der Dosierkapillare (110); und
- Aufteilung der Dosierkapillare (110) in mindestens zwei Teilstücke (160, 162), wobei mindestens eines der Teilstücke (160) das definierte Volumen des Zielbestandteils (164) enthält.

2. Verfahren nach dem vorhergehenden Anspruch, weiterhin umfassend ein Bereitstellen des definierten Volumens des Zielbestandteils (164) an mindestens eine medizinische und/oder diagnostische Verwendung, insbesondere ein Analyseverfahren zum Nachweis mindestens eines Analyten in dem Zielbestandteil (164).

3. Verfahren nach dem vorhergehenden Anspruch, wobei das Bereitstellen des definierten Volumens des Zielbestandteils (164) durch Annäherung einer Teilstücköffnung (114, 118, 170) mindestens eines der Teilstücke (160) an ein Testelement (166) und/oder einen Probenträger erfolgt.

4. Verfahren nach einem der vorhergehenden Ansprüche, wobei die Probe (134) eine Blutprobe umfasst, wobei bei der Bestandteilsseparation mittels Einwirken von Zentrifugalkräften und/oder Gravitationskräften korpuskuläre Bestandteile (152) der Probe (134) zumindest teilweise von Blutplasma (156) getrennt werden, wobei das Aufteilen der Dosierkapillare (110) derart erfolgt, dass das definierte Volumen des Zielbestandteils (164) ausschließlich Blutplasma (156) enthält.

5. Verfahren nach einem der vorhergehenden Ansprüche, wobei die Dosierkapillare (110) ein Kapillarvolumen aufweist, wobei das definierte Volumen des Zielbestandteils (164) weniger als 50% des Kapillarvolumens umfasst.

6. Verfahren nach einem der vorhergehenden Ansprüche, weiterhin umfassend mindestens einen nach dem Durchführen der Bestandteilsseparation und vor der Aufteilung der Dosierkapillare (110) durchgeführten Zwischenanalyseschritt, wobei aus der zumindest teilweisen Trennung der mindestens zwei Bestandteile der Probe (134) innerhalb der Dosierkapillare (110) auf mindestens eine Eigenschaft der Probe (134) geschlossen wird, insbesondere auf einen Anteil korpuskulärer Bestandteile in der Probe (134).

7. Verfahren nach einem der vorhergehenden Ansprüche, wobei mindestens eine der Öffnungen (112, 114), insbesondere eine distale Öffnung (116), eine Verengung (120) aufweist.

8. Verfahren nach einem der vorhergehenden Ansprüche, wobei mindestens eine der Öffnungen (112, 114), insbesondere eine proximale Öffnung (118), vor dem Durchführen der Bestandteilsseparation verschlossen wird, wobei das Verschließen vorzugsweise durch mindestens einen der folgenden Verschlüsse (146) erfolgt: ein Kitt; eine Kappe, insbesondere eine Kunststoff-Kappe, vorzugsweise eine Silikonkappe; ein Wachs, insbesondere ein Hämatokrit-Wachs; ein Harz; einen Klebstoff.

9. Verfahren nach einem der vorhergehenden Ansprüche, wobei zur Aufteilung der Dosierkapillare (110) mindestens ein mechanisches Bruchverfahren verwendet wird, wobei die Dosierkapillare (110) vorzugsweise mindestens eine Sollbruchstelle (124) aufweist.

10. Verfahren nach einem der vorhergehenden Ansprüche, wobei die Dosierkapillare (110) mindestens einen Antikoagulanz-Wirkstoff aufweist, insbesondere eine Antikoagulanz-Beschichtung (132), insbesondere eine EDTA-Beschichtung.

11. Dosierkapillare (110), insbesondere zur Verwendung in einem Verfahren nach einem der vorhergehenden Ansprüche, umfassend mindestens zwei Öffnungen (112, 114), wobei mindestens eine der Öffnungen (112, 114) mindestens eine Konstriktion (120) aufweist, wobei die Dosierkapillare (110) weiterhin zwischen den Öffnungen (112, 114) mindestens eine Trennlinie (122), insbesondere mindestens eine Trennlinie (122) mit mindestens einer Sollbruchstelle (124) und/oder mindestens einer Trennmarkierung, aufweist.

12. Vorrichtung zur Bereitstellung mindestens eines definierten Volumens eines Zielbestandteils (164) einer Probe (134), insbesondere unter Verwendung eines Verfahrens nach einem der vorhergehenden Verfahrensansprüche, umfassend mindestens eine Dosierkapillare (110), insbesondere nach dem vorhergehenden Anspruch, wobei die Dosierkapillare (110) mindestens zwei Öffnungen (112, 114) und mindestens eine Trennlinie (122) aufweist, insbesondere mindestens eine Trennlinie (122) mit mindestens einer Sollbruchstelle (124) und/oder mindestens einer Trennmarkierung, weiterhin umfassend mindestens eine Separationsvorrichtung (140) zum Durchführen einer Bestandteilsseparation zur zumindest teilweisen Trennung mindestens zweier Bestandteile (150, 154) der Probe (134) innerhalb der Dosierkapillare (110).

13. Vorrichtung nach dem vorhergehenden Anspruch, weiterhin umfassend eine Halterungsvorrichtung (138), welche eingerichtet ist, um die Dosierkapillare (110) in einer definierten Position für ein zumindest teilweises Befüllen der Dosierkapillare (110) mit der Probe (134) zu fixieren, insbesondere in einer im Wesentlichen waagerechten Position.
